# EUROPEAN PATENT APPLICATION

(11) **EP 4 613 775 A1**
(43) Date of publication of application: **10.09.2025**
(21) Application number: 24161991.5
(22) Date of filing: 07.03.2024
(51) Int. Cl.: C07K 16/30, A61K 39/395, A61P 35/00

(54) **TUMOR-SPECIFIC ANTIBODY**

(71) Applicant: Eberhard Karls Universität Tübingen Medizinische Fakultät, 72074 Tübingen (DE)
(72) Inventor: Kowarik, Markus Christian, 72076 Tübingen (DE); Schembecker, Sonja, 72076 Tübingen (DE)
(74) Representative: Witte, Weller & Partner Patentanwälte mbB

(57) **Abstract**

The present invention relates to an antibody or fragment thereof specifically binding to meningeosis carcinoma-specific antigen, such as DDX53, KIF3C, and AKR1A1, an antibody or fragment thereof for use in the prophylaxis and/or treatment of cancer, preferably melanoma, further preferably melanoma with leptomeningeal spread, highly preferably melanoma with meningeosis carcinomatosa, a pharmaceutical composition comprising said antibody or fragment thereof, a nucleic acid encoding said antibody or fragment thereof, a vector comprising said nucleic acid, a prokaryotic or eukaryotic host cell comprising said vector, and to a method for the prophylaxis and/or treatment of cancer, preferably melanoma, further preferably melanoma with leptomeningeal spread, highly preferably melanoma with meningeosis carcinomatosa, in a living being, comprising the administration of a prophylactically and/or therapeutically effective amount of said antibody or fragment thereof or said pharmaceutical composition.

## Description

The present invention relates to an antibody or fragment thereof specifically binding to meningeosis carcinoma-specific antigen, such as DDX53, KIF3C, and AKR1A1, an antibody or fragment thereof for use in the prophylaxis and/or treatment of cancer, preferably melanoma, further preferably melanoma with leptomeningeal spread, highly preferably melanoma with meningeosis carcinomatosa, a pharmaceutical composition comprising said antibody or fragment thereof, a nucleic acid encoding said antibody or fragment thereof, a vector comprising said nucleic acid, a prokaryotic or eukaryotic host cell comprising said vector, and to a method for the prophylaxis and/or treatment of cancer, preferably melanoma, further preferably melanoma with leptomeningeal spread, highly preferably melanoma with meningeosis carcinomatosa, in a living being, comprising the administration of a prophylactically and/or therapeutically effective amount of said antibody or fragment thereof or said pharmaceutical composition.

### BACKGROUND

Melanoma, also redundantly known as malignant melanoma, is a type of cancer that develops from the pigment-producing cells known as melanocytes.

Melanomas typically occur in the skin, but may rarely occur in the mouth, intestines, or eye. The primary cause of melanoma is ultraviolet light (UV) exposure in those with low levels of the skin pigment melanin. According to GLOBOCAN 2020, malignant melanoma was diagnosed in 324,635 people and caused 57,043 deaths in 2020. Melanoma is the most lethal skin cancer worldwide due to its high metastasis potential. Patients with metastatic melanoma have reduced survival compared with patients in earlier disease stages.

Chemotherapy drugs such as dacarbazine have been the backbone of metastatic melanoma treatment since FDA approval in 1975; however, its efficacy in terms of survival has never been proven in a randomized controlled trial.

Small-molecule targeted therapies work by blocking the genes involved in pathways for tumor proliferation and survival. The main treatments are BRAF, C-Kit and NRAS inhibitors. These inhibitors work to inhibit the downstream pathways involved in cell proliferation and tumor development due to specific gene mutations. However, melanoma tumors can develop resistance during therapy which can make therapy no longer effective.

An overview of the current options for adjuvant therapy for melanoma can be found in Lao et al. (2022), Current state of adjuvant therapy for melanoma: less is more, or more is better? Am. Soc. Clin. Oncol. Educ Book 42, 1-7.

Meningeosis carcinomatosa (MC) is a diffuse dissemination of tumor cells in the cerebrospinal fluid (CSF) and/or meninges and occurs in around 5 % of patients with malignant melanoma. The patients with such distant metastasis have a poor prognosis and are characterized by rapid disease progression and death from neurological causes.

Established treatments for singular brain metastases are neurosurgical resection and stereotactic radiotherapy, which can prolong survival. In patients with asymptomatic BRAF V600E-mutant brain metastases, the BRAF inhibitors dabrafenib, vemurafenib, and immunotherapy with ipilimumab are used. In the case of multiple symptomatic brain metastases, palliative whole-brain radiotherapy is used for treatment, although it has failed to show an overall survival benefit.

Overall, it can therefore be said that the treatment options for patients with MC in melanoma are extremely unsatisfactory.

### SUMMARY

Against this background, it is the object of the present invention to provide an alternative or new agent or compound with which the disadvantages of the prior art can be avoided or at least reduced. In particular, such an agent or compound is to be provided with which cancer, in particular melanoma, especially melanoma with leptomeningeal spread, such as meningeosis carcinomatosa (MC), can be prevented or treated in a targeted manner.

The problem underlying the invention is solved by the provision of an antibody or fragment thereof specifically binding to meningeosis carcinoma-specific antigen (MC-specific antigen), characterized in comprising
- as heavy chain variable domain a CDR3 region, said CDR3 region has an amino acid sequence being at least 90 % identical to one selected from those in SEQ ID NO: 1, 2 or 3, and/or
- as light chain variable domain a CDR3 region, said CDR3 region has an amino acid sequence being at least 90 % identical to one selected from those in SEQ ID NO: 4, 5 or 6.

The inventors were able to isolate B cells from the cerebrospinal fluid of melanoma patients with meningeosis carcinomatosa (MC), obtain antibodies from them and identify tumor-specific antibody binding sites. These tumor-specific antibody binding sites can be used to construct recombinant antibodies with a high therapeutic potential in an advantageous way.

In several melanoma patients with meningeosis, the inventors could generate a number of recombinant antibodies from clonally expanded B cells and detect tumor specificity of the antibodies. The recombinant antibodies were examined and verified with microarrays, immunofluorescence, flow cytometry and ELISA tests for tumor specificity.

The inventors' findings were not to be expected in particular because it is extremely difficult to isolate B cells or antibodies from body fluids that recognize certain tumor cells in a highly specific and selective manner, as - especially in the peripheral blood - latent infections of the individual from which the B cells originate cause a "background noise" by which the immune responses against the tumor are masked. Since the B cells are nearly absent in the cerebrospinal fluid under physiological conditions the inventors took advantage of the cerebrospinal fluid compartment being a separate immune compartment where B cells specifically maturated against the melanoma cells.

The term "antibody" is intended to include any polypeptide chain-containing molecular structure with a specific shape that fits to and recognizes an epitope, where one or more non-covalent binding interactions may stabilize the complex between the molecular structure and the epitope. The term includes both polyclonal and monoclonal antibodies. The archetypal antibody molecule is the immunoglobulin, and all types of immunoglobulins, IgG, IgM, IgA, IgE, IgD, etc., from all sources, e.g., human, rodent, rabbit, cow, sheep, pig, dog, camelid, other mammals, chicken, other avians, etc., are considered to be "antibodies". A preferred source for producing antibodies useful as starting material according to the invention is rabbits.

According to the invention, "CDR" refers to the complementary determining regions which, along with the framework regions (FR), are parts of the variable regions of the immunoglobulin and T cell receptor chains. CDRs and FRs are defined according to the International ImMunoGeneTics (IMGT) information system; see https://www.imgt.org/.

In an embodiment of the invention the antibody is a humanized antibody, i.e., an immunoglobulin or antibody that includes at least one humanized immunoglobulin or antibody chain (i.e., at least one humanized light or heavy chain). The term "humanized immunoglobulin chain" or "humanized antibody chain" (i.e., a "humanized immunoglobulin light chain" or "humanized immunoglobulin heavy chain") refers to an immunoglobulin or antibody chain (i.e., a light or heavy chain, respectively) having a variable region that includes a variable framework region substantially from a human immunoglobulin or antibody and complementarity determining regions (CDRs) (e.g., at least one CDR, preferably two CDRs, more preferably three CDRs) substantially from a non-human immunoglobulin or antibody, and further includes constant regions (e.g., at least one constant region or portion thereof, in the case of a light chain, and preferably three constant regions in the case of a heavy chain). The term "humanized variable region" (e.g., "humanized light chain variable region" or "humanized heavy chain variable region") refers to a variable region that includes a variable framework region substantially from a human immunoglobulin or antibody and complementarity determining regions (CDRs) substantially from a non-human immunoglobulin or antibody.

"Antibody fragments" comprise a portion of a full-length antibody, preferably the variable domain thereof, or at least the antigen binding site thereof, such as any of CDR3, CDR2 or CDR1. Examples of antibody fragments include diabodies, single-chain antibody molecules (scFv or scFab), and multispecific antibodies (e.g. bispecific) formed from antibody fragments.

The antibody or fragment thereof according to the invention can be produced by recombinant means. Methods for recombinant production are widely known in the state of the art and comprise protein expression in prokaryotic and eukaryotic cells with subsequent isolation of the antibody or fragment and usually purification to a pharmaceutically acceptable purity. For the expression of the antibodies or fragments as aforementioned in a host cell, nucleic acids encoding the respective light and/or heavy chains or fragments are inserted into expression vectors by standard methods. Expression is performed in appropriate prokaryotic or eukaryotic host cells like CHO cells, NSO cells, SP2/0 cells, HEK293 cells, COS cells, PER.C6 cells, yeast, or *E.coli* cells, and the antibody or fragment is recovered from the cells (supernatant or cells after lysis). General methods for recombinant production of antibodies are well-known in the state of the art and described, for example, in the review articles of Makrides, S.C. (1999), Protein Expr. Purif. 17, 183-202; Geisse et al. (1996), Protein Expr. Purif. 8, 271-282; Kaufman, R.J. (2000), Mol. Biotechnol. 16, 151-161; Werner, R.G. (1998), J. Drug Res. 48, 870-880.

As used herein, the term "binding" or "specifically binding" refers to the binding of the antibody or fragment thereof to an epitope of the antigen, e.g., with purified wild-type antigen in an *in vitro* assay such as a plasmon resonance assay (BIAcore, GE-Healthcare Uppsala, Sweden). Preferably, a specifically binding antibody or fragment does not exhibit significant cross-reactivity. An antibody or fragment thereof that "does not exhibit significant cross-reactivity" is one that will not appreciably bind to an undesirable entity, e.g., an undesirable proteinaceous entity. For example, an antibody or fragment that specifically bind to meningeosis carcinoma(MC)-specific antigen will appreciably bind MC-specific antigen but will not significantly react with non-MC-specific proteins or peptides, e.g., non-MC-specific proteins or peptides located in tumors. An antibody specific for a preferred epitope will, for example, not significantly cross-react with remote epitopes on the same protein or peptide. Specific binding can be determined according to any art-recognized means for determining such binding. Preferably, specific binding is determined according to Scatchard analysis and/or competitive binding assays. The affinity of the binding is defined by the terms ka (rate constant for the association of the antibody from the antibody/antigen complex), kD (dissociation constant), and KD (kD/ka). Binding or specifically binding means a binding affinity (KD) of 10⁻⁸ mol/l or less, preferably 10⁻⁹ M to 10⁻¹³ mol/l.

In an embodiment of the invention the antibody or fragment thereof is isolated. The term "isolated" means that the material is removed from its original environment (e.g., the natural environment, if it is naturally occurring). For example, a naturally occurring antibody or fragment thereof present in a living animal is not isolated, but the same antibody or fragment thereof, separated from some or all of the coexisting materials in the natural system, is isolated.

The inventors have recognized that, in one embodiment of the invention, the antibodies or fragments thereof need not necessarily be sequence identical with the indicated sequences, e.g., of the CDRs or CDR3 respectively. Specific and affine binding of the antibody or fragment is also possible if the binding regions are at least 90 % identical with the indicated specific sequences.

"Percent identity" or "percent identical" in turn, when referring to a sequence, means that a sequence is compared to a claimed or described sequence after alignment of the sequence to be compared (the "Compared Sequence") with the described or claimed sequence (the "Reference Sequence"). The percent identity is then determined according to the following formula: percent identity = 100 [1 -(C/R)]
wherein C is the number of differences between the Reference Sequence and the Compared Sequence over the length of alignment between the Reference Sequence and the Compared Sequence, wherein
(i) each amino acid or base in the Reference Sequence that does not have a corresponding aligned base or amino acid in the Compared Sequence and
(ii) each gap in the Reference Sequence and
(iii) each aligned amino acid or base in the Reference Sequence that is different from an aligned base or amino acid in the Compared Sequence, constitutes a difference and
(iv) the alignment has to start at position 1 of the aligned sequences;
and R is the number of amino acids or bases in the Reference Sequence over the length of the alignment with the Compared Sequence with any gap created in the Reference Sequence also being counted as an amino acid or base.

According to the invention, throughout the description and with respect to all embodiments, "at least 90 %" identity includes a sequence identity of 90, 91, 92, 93, 94, 95, 96, 97, 98, 99, and 100 %.

The antibody or fragment thereof disclosed in accordance with the present invention may also be in "purified" form. The term "purified" does not require absolute purity; rather, it is intended as a relative definition, and can include preparations that are highly purified or preparations that are only partially purified, as those terms are understood by those of skill in the relevant art. For example, individual clones isolated from cellular material have been conventionally purified to electrophoretic homogeneity. Purification of starting material or natural material to at least one order of magnitude, preferably two or three orders, and more preferably four or five orders of magnitude is expressly contemplated. Furthermore, a claimed antibody or fragment thereof which has a purity of preferably 99.999 %, or at least 99.99 % or 99.9 %; and even desirably 99 % by weight or greater is expressly encompassed.

The antibody or fragment thereof according to the invention may be in "enriched form". As used herein, the term "enriched" means that the concentration of the material is at least about 2, 5, 10, 100, or 1000 times its natural concentration (for example), advantageously 0.01 %, by weight, preferably at least about 0.1 % by weight. Enriched preparations of about 0.5 %, 1 %, 5 %, 10 %, and 20 % by weight are also contemplated. The sequences, constructs, vectors, cells, and other materials comprising the present invention can advantageously be in enriched or isolated form.

A "meningeosis carcinoma-specific antigen" or "MC-specific antigen" refers to an antigen that is specifically and selectively found in meningeosis carcinoma, preferably of human melanoma origin. Examples of such MC-specific antigens are the proteins DDX53, AKR1A1 and KIF3C.

These findings by the inventors were surprising, as the prior art does not describe the proteins DDX53, AKR1A1 and KIF3C as such specific tumor markers, in particular those associated with meningeosis carcinoma.

The testis/cancer antigen DDX53 also known as CAGE (cancer-associated gene) is an antigen that is expressed in various cancers but not in normal tissue except testis (Cho et al., Identification and characterization of a novel cancer/testis antigen gene CAGE. Biochem Biophys Res Commun. 2002, 292(3):715-26. The expression of DDX53 is related with the cell cycle suggesting that it might play a role in cellular proliferation (Cho et al., *ibid.*)*.* Another group showed co-expression of DDX53 with CD133, a marker for cancer stem cells and DDX53 directly regulated the SOX-2 expression as a factor for maintaining self-renewal in drug-resistant Malme3MR cells (Kim et al., DDX53 Regulates Cancer Stem Cell-Like Properties by Binding to SOX-2. Mol Cells. 2017, 40(5):322-330). DDX53 might therefore serve as an immunotherapeutic target for regulating cancer stem-like properties (Kim et al., *ibid*.). Another study showed that DDX53 promotes stem cell-like properties, autophagy, and confers resistance to anti-cancer drugs in breast cancer cells (Kim et al., 2017). DDX53 has also been shown to possess oncogenic potential and promotes cell cycle progression by inducing AP-1- and E2F-dependent expression of cyclins D1 and E (Por et al., The cancer/testis antigen CAGE with oncogenic potential stimulates cell proliferation by up-regulating cyclins D1 and E in an AP-1- and E2F-dependent manner. J Biol Chem. 2010, 285(19):14475-85).

Aldo-keto reductases (AKRs), belong to a highly conserved enzyme superfamily and are key enzymes for detoxification of reactive aldehydes. AKR1A1 has been shown to be widely distributed in cancer cells with relatively stable abundances and suggests involvement in tumorigenesis (Zhang et al., Quantitative analysis of the human AKR family members in cancer cell lines using the mTRAQ/MRM approach. J Proteome Res. 2013,12(5):2022-33). The AKR1A1 gene is also closely related to the prognosis of patients with lung adenocarcinoma (Liu et al., Identification of a novel glycolysis-related gene signature that can predict the survival of patients with lung adenocarcinoma. Cell Cycle. 2019,18(5):568-579) and has also been shown to be significantly overexpressed in breast cancer (Hlaváč et al., The role of cytochromes p450 and aldo-keto reductases in prognosis of breast carcinoma patients. Medicine (Baltimore). 2014, 93(28)).

KIF3C, a member of kinesin superfamily, was highly overexpressed in malignant melanoma (He et al., Identification of differentially expressed methylated genes in melanoma versus nevi using bioinformatics methods. Peer J. 2020, 8:e9273) and in breast cancer tissues also associated with tumor recurrence and lymph node metastasis (Wang et al., Suppression of motor protein KIF3C expression inhibits tumor growth and metastasis in breast cancer by inhibiting TGF-β signaling. Cancer Lett. 2015, 368(1):105-114). Silencing of KIF3C by shRNA inhibited epithelial-mesenchymal transition and metastasis by inhibiting TGF-β signaling and suppressed breast cancer cell proliferation (Wang et al., *ibid*.). Furthermore, KIF3C pathway promoted proliferation, migration, and invasion of glioma cells by activating the PI3K/AKT pathway (Gao et al., KIF3C promotes proliferation, migration, and invasion of glioma cells by activating the PI3K/AKT pathway and inducing EMT. Biomed Res Int. 2020, 6349312.).

The disclosed amino acid sequences have been identified by the inventors in such tumor- or MC-specific antibodies in the respective particular important CDR3 regions, in each case of the heavy and light chain, which are particularly suitable according to the invention to be applied prophylactically or therapeutically for the treatment of cancer, in particular melanoma.

In another embodiment of the invention said antibody or fragment thereof is further characterized in that
- the heavy chain variable domain comprises a CDR2 region, said CDR2 region has an amino acid sequence being at least 90 % identical to one selected from those in SEQ ID NO: 7, 8 or 9, and/or
- the light chain variable domain comprises a CDR2 region, said CDR2 region has an amino acid sequence being at least 90 % identical to one selected from those in SEQ ID NO: 10, 11, or 12.

In still another embodiment of the invention said antibody or fragment thereof is further characterized in that
- the heavy chain variable domain comprises a CDR1 region, said CDR1 region has an amino acid sequence being at least 90 % identical to one selected from those in SEQ ID NO: 13, 14, or 15, and/or
- the light chain variable domain comprises a CDR1 region, said CDR1 region has an amino acid sequence being at least 90 % identical to one selected from those in SEQ ID NO: 16, 17, or 18.

This measure provides the two further CDRs, i.e., CDR2 and CDR1, responsible for direct interaction with and binding to the antigen or epitope of the MC-specific antigen. By including these additional CDRs the affinity, specificity and selectivity and thus also prophylactic and therapeutic suitability of the antibody according to the invention and fragment thereof are thus further increased.

Another embodiment of the invention provides the antibody or fragment thereof which comprises
- the heavy chain variable domains
   - CDR3 region having an amino acid sequence being at least 90 % identical to said in SEQ ID NO: 1 and/or
   - CDR2 region having an amino acid sequence being at least 90 % identical to said in SEQ ID NO: 7 and/or
   - CDR1 region having an amino acid sequence being at least 90 % identical to said in SEQ ID NO: 13 and/or
- the light chain variable domains
   - CDR3 region having an amino acid sequence being at least 90 % identical to said in SEQ ID NO: 4 and/or
   - CDR2 region having an amino acid sequence being at least 90 % identical to said in SEQ ID NO: 10 and/or
   - CDR1 region having an amino acid sequence being at least 90 % identical to said in SEQ ID NO: 16.

With this measure, the antigen-binding sites responsible for antigen binding and therapeutic efficacy of the particular affine and MC-specific antibody designated by the inventors as 'clone P1-B4' are made available. Said clone and, thus, the antibody and fragment thereof according to this embodiment, specifically and selectively bind to protein DDX53. The antibody or fragment thereof according to this embodiment can be readily produced in a known manner.

Another embodiment of the invention provides the antibody or fragment thereof which comprises
- the heavy chain variable domains
   - CDR3 region having an amino acid sequence being at least 90 % identical to said in SEQ ID NO: 2 and/or
   - CDR2 region having an amino acid sequence being at least 90 % identical to said in SEQ ID NO: 8 and/or
   - CDR1 region having an amino acid sequence being at least 90 % identical to said in SEQ ID NO: 14 and/or
- the light chain variable domains
   - CDR3 region having an amino acid sequence being at least 90 % identical to said in SEQ ID NO: 5 and/or
   - CDR2 region having an amino acid sequence being at least 90 % identical to said in SEQ ID NO: 11 and/or
   - CDR1 region having an amino acid sequence being at least 90 % identical to said in SEQ ID NO: 17.

With this measure, the antigen-binding sites responsible for antigen binding and therapeutic efficacy of the particular affine and MC-specific antibody designated by the inventors as 'clone P1-F6' are made available. Said clone and, thus, the antibody and fragment thereof according to this embodiment, specifically and selectively bind to protein KIF3C. The antibody or fragment thereof according to this embodiment can be readily produced in a known manner.

Yet, another embodiment of the invention provides the antibody or fragment thereof which comprises
- the heavy chain variable domains
   - CDR3 region having an amino acid sequence being at least 90 % identical to said in SEQ ID NO: 3 and/or
   - CDR2 region having an amino acid sequence being at least 90 % identical to said in SEQ ID NO: 9 and/or
   - CDR1 region having an amino acid sequence being at least 90 % identical to said in SEQ ID NO: 15 and/or
- the light chain variable domains
   - CDR3 region having an amino acid sequence being at least 90 % identical to said in SEQ ID NO: 6 and/or
   - CDR2 region having an amino acid sequence being at least 90 % identical to said in SEQ ID NO: 12 and/or
   - CDR1 region having an amino acid sequence being at least 90 % identical to said in SEQ ID NO: 18.

With this measure, the antigen-binding sites responsible for antigen binding and therapeutic efficacy of the particular affine and MC-specific antibody designated by the inventors as 'clone P3-F1' are made available. Said clone and, thus, the antibody and fragment thereof according to this embodiment, specifically and selectively bind to protein AKR1A1. The antibody or fragment thereof according to this embodiment can be readily produced in a known manner.

In another embodiment of the invention said antibody or fragment thereof comprises
- a heavy chain having an amino acid sequence being at least 90 % identical to said in SEQ ID NO:19, and/or
- a light chain having an amino acid sequence being at least 90 % identical to said in SEQ ID 20.

In this embodiment, the entire amino acid sequences of the hypervariable regions of the heavy (IgH) and light chains (IgK) of the antibody of the invention (clone P1-B4; target DDX53) are provided, i.e., not only the sequences of CDR1, 2, and 3, but also the sequences of the intervening 'framework regions' (FRs) FR1, FR2, FR3, and FR4. The production is thus considerably simplified, and the resulting antibody and fragment are characterized by particular suitability according to the invention for the prophylaxis and treatment of cancer and in particular melanoma.

In another embodiment of the invention the antibody or fragment thereof comprises
- a heavy chain having an amino acid sequence being at least 90 % identical to said in SEQ ID NO:21, and/or
- a light chain having an amino acid sequence being at least 90 % identical to said in SEQ ID 22.

In this embodiment, the entire amino acid sequences of the hypervariable regions of the heavy (IgH) and light chains (IgK) of the antibody of the invention (clone P1-F6; target KIF3C) are provided, i.e., not only the sequences of CDR1, 2, and 3, but also the sequences of the intervening 'framework regions' (FRs) FR1, FR2, FR3, and FR4. The production is thus considerably simplified, and the resulting antibody and fragment are characterized by particular suitability according to the invention for the prophylaxis and treatment of cancer and in particular melanoma.

In yet another embodiment of the invention the antibody or fragment thereof comprises
- a heavy chain having an amino acid sequence being at least 90 % identical to said in SEQ ID NO:23, and/or
- a light chain having an amino acid sequence being at least 90 % identical to said in SEQ ID 24.

In this embodiment, the entire amino acid sequences of the hypervariable regions of the heavy (IgH) and light chains (IgK) of the antibody of the invention (clone P3-F1; target AKR1A1) are provided, i.e., not only the sequences of CDR1, 2, and 3, but also the sequences of the intervening 'framework regions' (FRs) FR1, FR2, FR3, and FR4. The production is thus considerably simplified, and the resulting antibody and fragment are characterized by particular suitability according to the invention for the prophylaxis and treatment of cancer and in particular melanoma.

In an embodiment of the invention said antibody or fragment thereof specifically bind to the MC-specific antigen, which is selected from the group consisting of DDX53, KIF3C, and AKR1A1.

Since no antibody is yet available in the prior art, which specifically and selectively binds to MC-specific antigen, and which is suitable for a prophylactic and/or therapeutic use in medicine, especially in the treatment of cancer and in particular melanoma, a further object of the invention relates precisely to such an antibody or fragment thereof configured for a specific binding to MC-specific antigen, preferably DDX53, KIF3C, and AKR1A1. Further preferably, this antibody or fragment thereof are the antibody or fragment thereof according to the invention, which is described in detail above.

The features, characteristics, advantages and embodiments mentioned further above apply *mutatis mutandis* also for this subject-matter.

As used herein, "prophylaxis" describes the totality of all measures taken for this purpose to prevent impairment of health by risk factors, diseases or accidents. The prevention of secondary diseases or maldevelopments by timely treatment of a primary disease is also a form of prophylaxis. In relation to the invention is thought, in particular, to the prevention of the development of cancer, especially melanoma.

Prophylaxis of cancer also includes "prevention of metastasis" or "prevention of secondary tumors", i.e., measures aiming to prevent the transmission of cancerous cells from the primary tumor to one or more sites elsewhere in a patient where then secondary tumors develop. This means that the metastasis of the primary, tumor or cancer is prevented, delayed, or reduced and thus the development of secondary tumors is prevented, delayed, or reduced. Preferably the metastasis, i.e., secondary tumors, of the lung are prevented or reduced, which means that metastatic transmission of cancerous cells from the primary tumor to the lung is prevented or reduced.

According to the invention "treatment", as used in this context, refers to therapeutic measures aimed either at eliminating the cause of the disease (causal therapy) or at eliminating the symptoms (symptomatic therapy). The invention, in particular, refers to the treatment of cancer, especially melanoma. Also included is the use of the antibody or fragment thereof according to the invention for use in adjuvant therapy. Adjuvant therapy generally refers to supplementary or supportive therapeutic measures, but in particular after surgical removal of the tumor, e.g., melanoma.

Another subject-matter of the invention relates to an antibody or fragment thereof, which specifically bind to MC-specific antigen, characterized in that it specifically binds to an epitope comprising an amino acid sequence being at least 90 % identical to one selected from those in SEQ ID NOS: 49-55.

Herein, a further antibody or fragment thereof according to the invention is provided which can specifically bind to the epitopes of the new tumor markers recognized for the first time by the inventors. The amino acid sequences SEQ ID Nos: 49, 50 and 51 form epitopes of the target protein DDX5. The amino acid sequences SEQ ID Nos: 52 and 53 form epitopes of the target protein KIFC3. The amino acid sequences SEQ ID Nos: 54 and 55 form epitopes of the target protein AKR1A1. Thereby, "specific binding" means that the antibody or fragment selectively bind to the epitopes mentioned under stringent conditions, but not to other epitopes or targets.

A yet further subject-matter of the invention relates to a polypeptide comprising an amino acid sequence being at least 90 % identical to one selected from those in SEQ ID NOS: 49-55.

Such a polypeptide can be used as an active ingredient of a peptide vaccine, aiming at inducing an immune response capable of treating and/or preventing a tumor, such as a melanoma, preferably melanoma with meningeosis carcinomatosa (MC). In another approach such a polypeptide can be used to generate CAR T cells specifically binding to the epitopes identified by the inventors.

Still another subject-matter of the invention relates to a pharmaceutical composition characterized in comprising an antibody of fragment thereof or a polypeptide according to the invention.

The features, characteristics, advantages and embodiments mentioned in relation with the antibody or fragment thereof apply *mutatis mutandis* also for the pharmaceutical composition.

The pharmaceutical composition may comprise a pharmaceutical carrier. As used herein, "pharmaceutical carrier" includes any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (e.g. by injection or infusion).

A pharmaceutical composition of the present invention can be administered by a variety of methods known in the art. As will be appreciated by the skilled artisan, the route and/or mode of administration will vary depending upon the desired results. To administer a compound of the invention by certain routes of administration, it may be necessary to coat the compound with, or co-administer the compound with, a material to prevent its inactivation. For example, the compound may be administered to a subject in an appropriate carrier, for example, liposomes, or a diluent. Pharmaceutically acceptable diluents include saline and aqueous buffer solutions. Pharmaceutical carriers include sterile aqueous solutions or dispersions and sterile powders for the extemporaneous preparation of sterile injectable solutions or dispersion. The use of such media and agents for pharmaceutically active substances is known in the art.

These pharmaceutical compositions may also contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol, sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Actual dosage levels of the active ingredient, i.e., antibody or fragment thereof or polypeptide, in the pharmaceutical composition of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

In an embodiment the antibody and/or fragment thereof or polypeptide are the only active ingredients of the pharmaceutical composition. In another embodiment, the pharmaceutical composition may contain additional active agents, such as anti-cancer or anti-melanoma compounds, e.g., traditional chemotherapeutics, interferons, imatinib, anti-PD-1 antibodies, BRAF and/or MEK inhibitors, etc. The pharmaceutical composition comprising the polypeptide according to the invention can be an anti-tumor vaccine and, in an embodiment, may contain an adjuvant as activity enhancer.

A still further subject-matter according to the invention relates to a kit comprising:
a) a container comprising the antibody or fragment thereof according to the invention in solution or in lyophilized formulation;
b) optionally, a second container containing a diluent or reconstituting solution for the lyophilized formulation;
c) optionally, instructions for (i) use of the solution or (ii) reconstitution and/or use of the lyophilized formulation.

The kit may further comprise one or more of (iii) a buffer, (iv) a diluent, (v) a filter, (vi) a needle, or (v) a syringe. The container is preferably a bottle, a vial, a syringe or test tube; and it may be a multi-use container. The pharmaceutical composition is preferably lyophilized.

The kit of the present invention preferably comprises a lyophilized formulation of the present invention in a suitable container and instructions for its reconstitution and/or use. Suitable containers include, for example, bottles, vials (e.g., dual chamber vials), syringes (such as dual chamber syringes) and test tubes. The container may be formed from a variety of materials such as glass or plastic. Preferably the kit and/or container contain/s instructions on or associated with the container that indicates directions for reconstitution and/or use. For example, the label may indicate that the lyophilized formulation is to be reconstituted to peptide concentrations as described above. The label may further indicate that the formulation is useful or intended for subcutaneous administration.

The features, characteristics, advantages and embodiments mentioned in relation with the antibody or fragment thereof apply *mutatis mutandis* also to the kit.

Still another subject-matter of the invention relates to a nucleic acid encoding an antibody or fragment thereof or a polypeptide according to the invention.

This measure allows the recombinant production of the antibody or fragment thereof or polypeptide, according to the invention, in an advantageous manner. Methods for recombinant production are widely known in the state of the art as has been described further above.

The features, characteristics, advantages and embodiments mentioned in relation with the antibody or fragment thereof apply *mutatis mutandis* also for the nucleic acid.

The terms "nucleic acid" or "nucleic acid molecule", as used herein interchangeably, are intended to include DNA molecules and RNA molecules. A nucleic acid molecule may be single-stranded or double-stranded, but preferably is double-stranded DNA. It includes oligonucleotide molecules.

The nucleic acid molecule or oligonucleotide according to the invention, in an embodiment, comprise a nucleotide sequence being at least 90 % identical to one selected from those in SEQ ID NO: 25, 27, 28, 29, 30, 31, 32, 33, 34, 35, 36, 37, 38, 39, 40, 41, 42, 43, 44, 45, 46, 47, and/or 48.

Yet, another subject-matter of the present invention is a vector, preferably an expression vector, comprising the nucleic acid or oligonucleotide according to the invention, and, optionally, regulatory elements necessary for expression in a prokaryotic and/or eukaryotic cell.

A "vector" is a nucleic acid molecule, in particular self-replicating, which transfers an inserted nucleic acid molecule into and/or between host cells. The term includes vectors that function primarily for insertion of DNA or RNA into a cell (e.g., chromosomal integration), replication of vectors that function primarily for the replication of DNA or RNA, and expression vectors that function for transcription and/or translation of the DNA or RNA. Also included are vectors that provide more than one of the functions as described.

An "expression vector" is a polynucleotide which, when introduced into an appropriate host cell, can be transcribed and translated into a polypeptide. An "expression system" usually refers to a suitable host cell comprised of an expression vector that can function to yield a desired expression product. "Regulatory elements" of expression vectors are well known to the skilled artisan and include, e.g., promotors, enhancers, polyadenylation signals and transcription termination sequence, etc.

The features, characteristics, advantages and embodiments mentioned in relation with the antibody or fragment thereof apply *mutatis mutandis* also for the vector.

Still another subject-matter of the invention is a prokaryotic or eukaryotic host cell comprising the vector according to the invention.

The term "host cell" as used in the current application denotes any kind of cellular system which can be engineered to generate the antibodies according to the current invention. "Host cell" herein typically refers to a cell or non-human organism genetically engineered to produce the antibodies or fragments thereof according to the invention. These hosts and host cells include mammalian cells, bacterial, yeast, insect cells, plant cells and transgenic plants or animals such as rodents, plants and bovines. Typically, antibodies or antibody fragments are expressed in mammalian, bacterial and yeast cells. In one embodiment HEK293 cells and CHO cells are used as host cells.

The features, characteristics, advantages and embodiments mentioned in relation with the antibody or fragment thereof apply *mutatis mutandis* also for the prokaryotic or eukaryotic host cell.

Another subject-matter of the invention relates to a method for the prophylaxis and/or treatment of cancer, preferably melanoma, in a living being, comprising the administration of a prophylactically and/or therapeutically effective amount of the antibody or fragment thereof or polypeptide and/or the pharmaceutical composition according to the invention.

The features, characteristics, advantages and embodiments mentioned in relation with the antibody or fragment thereof apply *mutatis mutandis* also for the method according to the invention.

A "living being" refer to any subject or organism, including mammals, in particular humans.

The invention is now further explained by means of embodiments and examples resulting in additional features, characteristics and advantages of the invention. The embodiments and examples are of pure illustrative nature and do not limit the scope or range of the invention. The features mentioned in the specific embodiments and examples are features of the invention and may be seen as general features which are not applicable in the specific embodiment or example but also in an isolated manner in the context of any embodiment or example of the invention.

The invention is now further described and explained in more detail by referring to the following non-limiting examples and figures.

### BRIEF DESCRIPTION OF THE FIGURES

- Figure 1:: Immunohistochemistry staining with fixed Malme-3M melanoma cells and the recombinant antibodies P1-B4, P1-B8, P3-F1 and P1-F6. Additionally, the proliferation marker Ki-67 and DAPI was stained.
- Figure 2:: Binding of recombinant antibodies to selected targets tested by custom ELISA. Additionally, serum and liquor supernatant of the respective patients was tested. Optical density (OD) was measured at 450 nm. ODs above 0.1 were considered as positive for binding.
- Figure 3:: Predicted binding clusters of each antibody with its respective potential target using AbAdapt. The first five top hits of the antibody binding are shown. The Top hits are colored in the following order beginning with cluster 0 (highest rate) red, blue, yellow, magenta and cyan. The respective target is displayed in grey.

### EXAMPLES

### 1. Introduction

The concept of immuno-surveillance in humans was first described in the late 1950s by Thomas and Burnet who suggested that lymphocytes act as sentinels that continuously eliminate neo-transformed cells to prevent manifestation of neoplasms. Decades later, this theory has been widely accepted and the use of checkpoint inhibitors has revolutionized treatment strategies in cancer.

Regarding the role of lymphocytes during carcinogenesis, T cells have been in the focus of research whereas systemic studies on B cells are scarce. Most studies on B cell infiltration and tumor specific antibody production in humans concentrated on tumors outside the CNS with partially controversial results. Whereas several studies found correlations between tumor B cell infiltrates and favorable clinical outcomes, other results suggested tumor promoting roles of B cells. Concerning CNS tumors, studies on B cell infiltration and the cerebrospinal fluid (CSF) in cancer patients with gliomas, brain or meningeal metastasis are generally scarce and the role of B cells is poorly understood. Detailed studies on the humoral immune reaction showed that tumor specific antibodies can be identified in the peripheral blood and tumor tissues of cancer patients. A major drawback has been that the analysis of blood-derived B cells is highly susceptible to "background noise" from latent infections and the immune response to everyday pathogens, which can mask acute immune responses against the tumor. As a result, clonally expanded B cells in peripheral blood do not usually target malignant tissue, and the search for peripheral tumor-specific B cells resembles the proverbial search for a needle in a haystack.

In a previous study it was shown that elevated B-cell levels in the CSF are detectable in a subset of patients with CNS neoplasia, including brain metastases and meningeal carcinomatosis. B cells are largely absent from the CSF of healthy individuals, and an elevated CSF B cell fraction is usually associated with defined immune responses. Furthermore, CSF B cell derived recombinant antibodies in several diseases such as subacute sclerosing panencephalitis, multiple sclerosis and neuromyelitis optica spectrum disorder have been shown to be disease relevant. Taking advantage of the CNS as a separate compartment, the present invention aims to determine whether the observed CSF B cell population in patients with meningeal carcinomatosis represents a specific response against tumor cells.

In order to further analyze the CSF B cell reaction during meningeal carcinomatosis, three melanoma patients with leptomeningeal spread and an elevated B cell fraction were investigated. By applying single-cell sequencing, paired heavy and light chain immunoglobulin sequences were recovered, 13 recombinant antibodies were generated and tested for tumor specificity. This proof-of-concept study demonstrates antibodies binding to defined targets, identifies potential epitopes of these targets, and presents compelling evidence for a tumor-targeted B cell response in melanoma patients with leptomeningeal spread.

### 2. Methods

Three melanoma patients with leptomeningeal spread and a presumable CSF B cell reaction were selected by analyzing CSF standard parameters including intrathecal immunoglobulin (Ig) synthesis. Single cell sorting of CSF B cells was performed followed by conventional sequencing of Ig heavy and light chain transcripts. 13 recombinant antibodies were generated from CSF B cells and were tested for antigen specificity using antigen microarrays, ELISA and Immunocytochemistry as well as bioinformatic tools for binding predictions.

### 3. Results

Single cell immunoglobulin repertoires were assessed in all three selected patients. On average, 46 heavy chain (range 24 - 73) and 41 light chain (range 13 - 75) sequences were recovered. In total, 60 paired heavy and light chain sequences were found in patient 1, 32 in patient 2 and 10 in patient 3. The average assignment of sequences to clonal groups was 61 % for heavy chain (range 34 % - 77 %) and 52 % for light chain sequences (range 28 % - 73 %). The absolute number of clones was 7 in patient 1 and 3 for each patient 2 and patient 3 (Table 1). Clonal groups showed several mutations to germline which is indicative for ongoing somatic hypermutations.

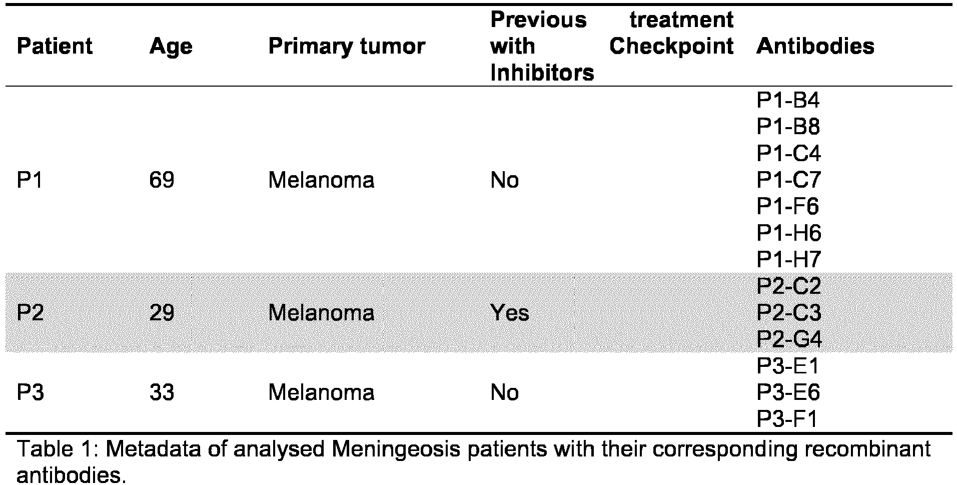

### Antigen recognition and epitope mapping by microarrays

In order to identify the potential target antigens of the produced recombinant antibodies, pooled antibodies (batch 1: 7 antibodies from patient 1; batch 2: 6 antibodies from patients 2 and 3) were screened by the HuProt^{™} Human Proteome Microarray v4.0 (PEPperPRINT). A prominent cut-off after the first two proteins (AKR1A1, GART) was detectable in antibody batch 2 from patients 2 and 3 (Table 3) so that those were considered to be the most likely candidate target antigens for these antibodies. Since the cut-off for antibody batch 1 of patient 1 was not as clear (Table 2), the first 50 protein hits were further analyzed according to similarity, biological function and tumor association. Within these first hits three peptidyl prolyl isomerases (PPIL1, PPIE, PPIA) and two phosphatases (PPP1R12B, MDP1) were identified. Both types of enzymes are heavily involved in oncogenic processes. In addition, 6 antigens (LASP1, MTUS2, CCR1, KIFC3, LMNA, DDX53) were found to be listed as either cancer enhanced or cancer enriched in the Human Protein Atlas.

**Table 2: Top 50 hits of HuProt^{™} Human Proteome Microarray v4.0 of antibody pool from patient 1. Selected proteins for further analyses are highlighted.**

| **Protein** | **UniProt ID** | **Full Name** | **Corrected Intensity Ratio** |
|---|---|---|---|
| PPP1R12B | 060237-2 | protein phosphatase 1 regulatory subunit 12B | 1309.2 |
| **PPIL1** | Q9Y3C6 | peptidylprolyl isomerase like 1 | 1309.2 |
| SHFM1 | P60896 | SEM1 26S proteasome complex subunit | 1309.1 |
| RAD23A | P54725-3 | RAD23 homolog A, nucleotide excision repair protein | 1309.1 |
| **LASP1** | Q14847 | LIM and SH3 protein 1 | 1309.0 |
| CCR1 | P32246 | C-C motif chemokine receptor 1 | 1308.6 |
| RAD23A | P54725 | RAD23 homolog A, nucleotide excision repair protein | 1304.6 |
| PPIE | Q9UNP9 | peptidylprolyl isomerase E | 1297.1 |
| NACA | Q13765 | nascent polypeptide associated complex subunit alpha | 1295.7 |
| SETD7 | Q8WTS6 | SET domain containing 7, histone lysine methyltransferase | 1288.3 |
| APBB1 | 000213 | amyloid beta precursor protein binding family B member 1 | 1287.9 |
| ACBD3 | Q9H3P7 | acyl-CoA binding domain containing 3 | 1269.5 |
| WASF2 | Q9Y6W5 | WASP family member 2 | 1206.0 |
| PPIA | P62937 | peptidylprolyl isomerase A | 1205.9 |
| UBL7 | Q96S82 | ubiquitin like 7 | 1203.9 |
| GPBP1_frag | | GC-rich promoter binding protein 1 | 1175.1 |
| NAPB | Q9H115 | NSF attachment protein beta | 1172.1 |
| DBNL | Q9UJU6 | drebrin like | 1081.8 |
| **MTUS2** | Q5JR59-3 | microtubule associated scaffold protein 2 | 1068.7 |
| KCTD5 | Q9NXV2 | potassium channel tetramerization domain containing 5 | 1051.5 |
| LRRFIP1 | Q32MZ4 | LRR binding FLII interacting protein 1 | 1048.3 |
| SCL-70 | | autoantigen | 1007.5 |
| CCDC102B_frag | | coiled-coil domain containing 102B | 984.8 |
| **KIFC3** | Q9BVG8-5 | kinesin family member C3 | 942.5 |
| **MDP1** | Q86V88 | magnesium dependent phosphatase 1 | 919.9 |
| DBNL | Q9UJU6-2 | drebrin like | 918.3 |
| PEX19 | P40855 | peroxisomal biogenesis factor 19 | 917.0 |
| SF3A1 | Q15459 | splicing factor 3a subunit 1 | 914.2 |
| CALCOCO2 | Q13137 | calcium binding and coiled-coil domain 2 | 899.4 |
| **LMNA** | | lamin A/C | 894.4 |
| SLC7A6OS | Q96CW6 | solute carrier family 7 member 6 opposite strand | 894.0 |
| ACRC | Q96QF7 | germ cell nuclear acidic peptidase | 893.9 |
| Hep B Protein X | | autoantigen | 865.0 |
| **DDX53** | Q86TM3 | DEAD-box helicase 53 | 864.5 |
| **ACY3** | Q96HD9 | aminoacylase 3 | 863.6 |
| CRK | P46108 | CRK proto-oncogene, adaptor protein | 821.9 |
| TEX13A | Q9BXU3 | testis expressed 13A | 819.2 |
| PARVA | Q9NVD7 | parvin alpha | 816.2 |
| PA2G4 | Q9UQ80 | proliferation-associated 2G4 | 776.7 |
| CEP85 | Q6P2H3-3 | centrosomal protein 85 | 730.4 |
| BRD2 | P25440 | bromodomain containing 2 | 706.4 |
| NACA2 | Q9H009 | nascent polypeptide associated complex subunit alpha 2 | 701.7 |
| PSMD7 | P51665 | proteasome 26S subunit, non-ATPase 7 | 683.1 |
| GIPC1 | 014908-2 | GIPC PDZ domain containing family member 1 | 666.3 |
| CABP4 | P57796-2 | calcium binding protein 4 | 651.7 |
| KIFC3 | Q9BVG8-5 | kinesin family member C3 | 646.1 |
| SMARCC1 | Q92922 | SWI/SNF related, matrix associated, actin dependent regulator of chromatin subfamily c member 1 | 592.2 |
| PACSIN2 | Q9UNF0 | protein kinase C and casein kinase substrate in neurons 2 | 591.9 |
| ASCC2 | Q9H1I8 | activating signal cointegrator 1 complex subunit 2 | 580.5 |
| OTUD6B | Q8N6M0 | OTU domain containing 6B | 577.6 |

**Table 3: Top 50 hits of HuProt^{™} Human Proteome Microarray v4.0 of antibody pool from patient 2 and patient 3. Selected proteins for further analyses are highlighted.**

| **Protein** | **UniProt ID** | **Full Name** | **Corrected Intensity Ratio** |
|---|---|---|---|
| **AKR1A1** | P14550 | aldo-keto reductase family 1 member A1 | 1309.3 |
| **GART** | P22102-2 | phosphoribosylglycinamide formyltransferase, phosphoribosylglycinamide synthetase, phosphoribosylaminoimidazole synthetase | 1308.9 |
| RHOA | P61586 | ras homolog family member A | 786.9 |
| SHFM1 | P60896 | SEM1 26S proteasome complex subunit | 718.2 |
| RAC3 | P60763 | Rac family small GTPase 3 | 715.4 |
| RAB11A | P62491 | RAB11A, member RAS oncogene family | 709.4 |
| SNX9 | Q9Y5X1 | sorting nexin 9 | 638.4 |
| OFD1_frag | | OFD1 centriole and centriolar satellite protein | 606.5 |
| NAPB | Q9H115 | NSF attachment protein beta | 582.7 |
| SLC7A6OS | Q96CW6 | solute carrier family 7 member 6 opposite strand | 548.9 |
| OFD1_frag | | OFD1 centriole and centriolar satellite protein | 519.2 |
| RAP1GDS1 | P52306-2 | Rap1 GTPase-GDP dissociation stimulator 1 | 506.6 |
| ING3 | Q9NXR8-2 | inhibitor of growth family member 3 | 471.9 |
| PMEPA1 | Q969W9-2 | prostate transmembrane protein, androgen induced 1 | 442.0 |
| XRCC4 | Q13426 | X-ray repair cross complementing 4 | 440.7 |
| CCDC102B_frag | | coiled-coil domain containing 102B | 438.4 |
| ACSL4 | O60488-2 | acyl-CoA synthetase long chain family member 4 | 403.1 |
| ARFGAP1 | Q8N6T3-2 | ADP ribosylation factor GTPase activating protein 1 | 377.3 |
| MED22 | Q15528-2 | mediator complex subunit 22 | 362.8 |
| USP5 | P45974 | ubiquitin specific peptidase 5 | 360.9 |
| PPM1B | O75688-2 | protein phosphatase, Mg2+/Mn2+ dependent 1B | 348.5 |
| RAC1 | P63000 | Rac family small GTPase 1 | 323.9 |
| PPM1J | Q5JR12 | protein phosphatase, Mg2+/Mn2+ dependent 1J | 312.9 |
| NAP1L1 | P55209 | nucleosome assembly protein 1 like 1 | 312.6 |
| XRCC4 | Q13426-3 | X-ray repair cross complementing 4 | 292.5 |
| ZFYVE16 | Q7Z3T8-3 | zinc finger FYVE-type containing 16 | 289.2 |
| TLE3 | Q04726 | TLE family member 3, transcriptional corepressor | 283.5 |
| PPM1D | 015297-2 | protein phosphatase, Mg2+/Mn2+ dependent 1D | 272.7 |
| ARFGAP1 | Q8N6T3 | ADP ribosylation factor GTPase activating protein 1 | 272.6 |
| APBB1 | 000213 | amyloid beta precursor protein binding family B member 1 | 269.8 |
| ARHGEF16 | Q5VV41-2 | Rho guanine nucleotide exchange factor 16 | 268.6 |
| PDCL | Q13371 | phosducin like | 267.9 |
| TSEN15 | Q8WW01 | tRNA splicing endonuclease subunit 15 | 241.3 |
| SCYL3 | Q8IZE3-2 | SCY1 like pseudokinase 3 | 236.9 |
| RBBP7 | Q16576 | RB binding protein 7, chromatin remodeling factor | 235.5 |
| CSNK1G1 | Q9HCP0-2 | casein kinase 1 gamma 1 | 221.6 |
| PVRL3_frag | | nectin cell adhesion molecule 3 | 210.7 |
| C14orf37 | Q86TY3 | armadillo like helical domain containing 4 | 210.2 |
| RAB2B | Q8WUD1 | RAB2B, member RAS oncogene family | 208.8 |
| EEF2K | 000418 | eukaryotic elongation factor 2 kinase | 208.7 |
| USP5 | P45974-2 | ubiquitin specific peptidase 5 | 200.6 |
| LDLRAD4 | 015165-7 | low density lipoprotein receptor class A domain containing 4 | 196.9 |
| AKT3 | Q9Y243-2 | AKT serine/threonine kinase 3 | 195.5 |
| RAC1 | P63000 | Rac family small GTPase 1 | 192.5 |
| LOC105372277 | | Ensembl ID: ENSP00000394047 | 188.9 |
| KRT8 | | keratin 8 | 187.8 |
| CALU | O43852 | calumenin | 187.7 |
| MDP1 | Q86V88 | magnesium dependent phosphatase 1 | 185.4 |
| GPBP1_frag | | GC-rich promoter binding protein 1 | 185.4 |
| PRDM4 | Q9UKN5 | PR/SET domain 4 | 179.0 |

### Selected recombinant antibodies present binding to the melanoma cell line Malme-3M

Flow cytometry was performed with all 13 recombinant antibodies against the Malme-3M cell line to evaluate general binding properties to melanoma cells. Since the potential targets detected were intracellular proteins, cells were permeabilized. The recombinant antibodies P1-F6 presented positive staining (38 % positive cells), while all other antibodies were considered as negative (0 - 1 % positive cells). Interestingly, when testing non-permeabilized Malme-3M cells not only P1-F6 but also P1-B4, P1-B8 and P3-F1 showed a huge proportion of cells bound to the respective antibody (78.0 - 99.6 % positive cells). Additionally, the three antibodies P3-E6, P1-H6 and P1-H7 presented a small but not neglectable proportion of bound cells (21.8 - 33.9 % positive cells). These results indicate an extracellular binding of the antibodies. One can hypothesise that these target proteins are presented on MHC I and induce a T-cell directed B-cell activation and antibody production against them.

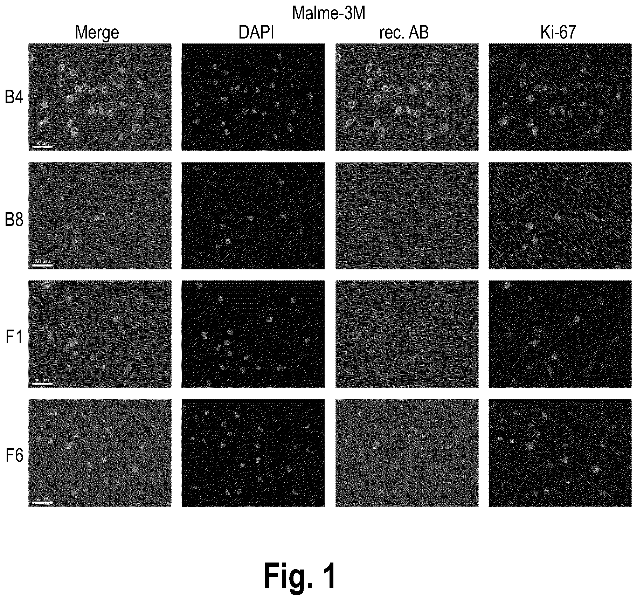

Antibodies that bound to Malme-3M cells in the flow cytometric experiments were further analyzed in fixed Malme-3M cells to further evaluate the binding patterns. Intracellular binding mostly found on the edges of the cells was observed for all four antibodies tested, even though P1-B8 and P3-F1 presented only weak staining (Figure 1). P1-B4 had the most intense staining. Co-staining with KI-67, a marker for cell proliferation, was also performed which mostly showed intra-nuclear staining patterns. In co-staining with our recombinant antibodies and KI-67, the inventors observed a partial overlap at the border of cell nuclei.

### Confirming DDX53, KIFC3 and AKR1A1 as antibody targets

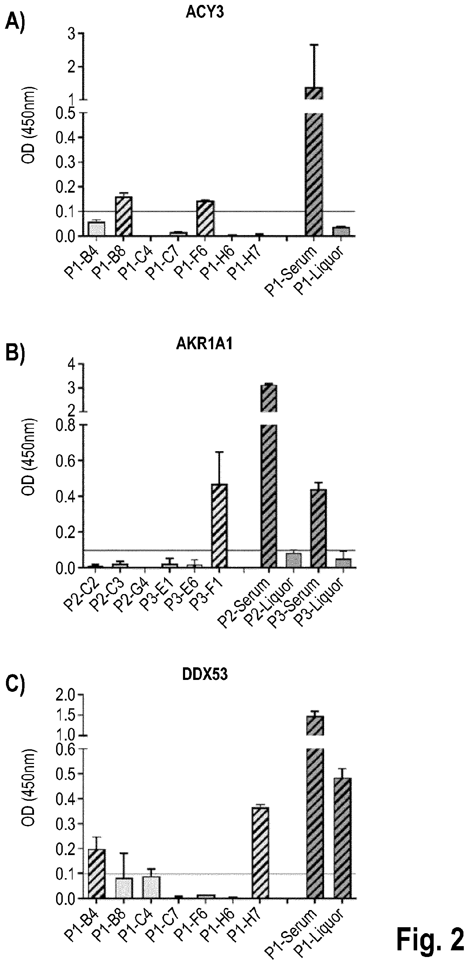

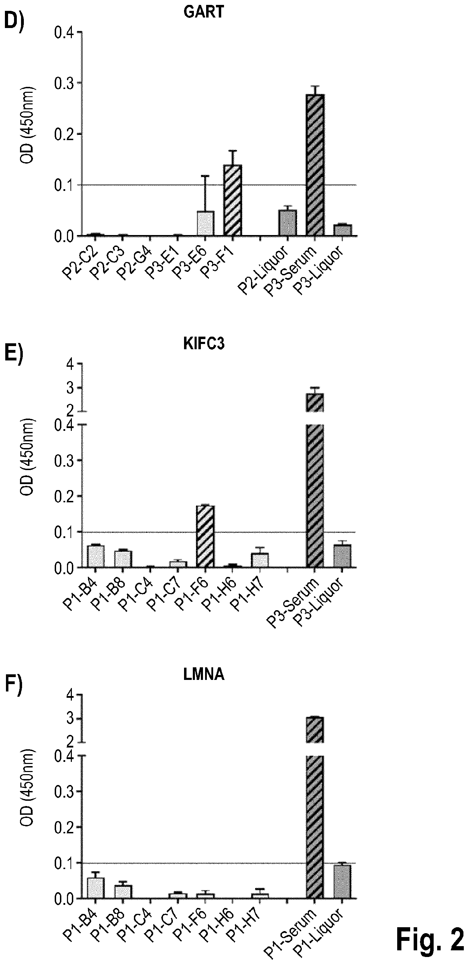

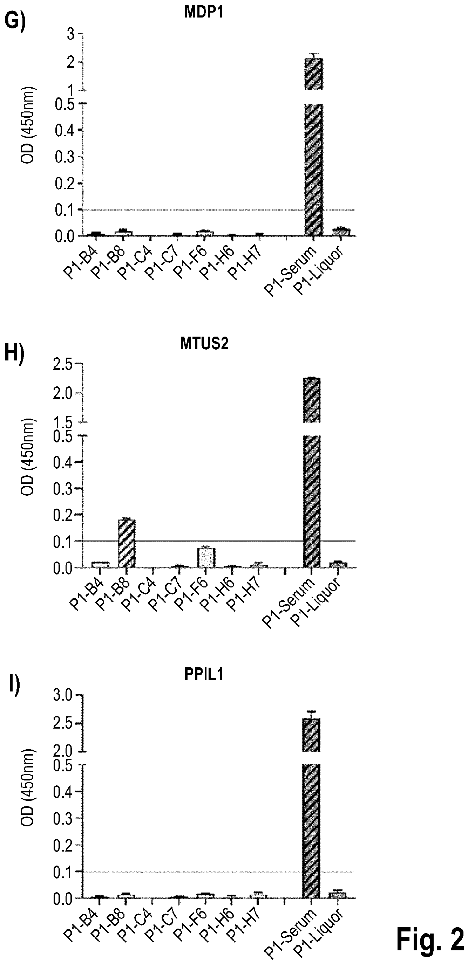

To validate the possible antigen targets found by the microarrays, custom ELISAs were performed to test the binding of the recombinant antibodies to ACY3, AKR1A1, DDX53, GART, KIFC3, LMNA, MDP1, MTUS2 and PPIL1 (Figure 2). Additionally, also serum and liquor supernatant of the respective patients was tested. Two antibodies (P1-B4 and P1-H7) from patient 1 were found to bind the known tumor-associated DDX53 protein. Also, serum and liquor from this patient were tested positive. Besides this, P3-F1 also binds only one target, AKR1A1. In this case only the serum but not the liquor of this patient was positive. The antibodies P1-F6 and P1-B8 from patient 1 were found to bind to KIFC3 and ACY3 as well as ACY3 and MTUS2, respectively. Probably those two are rather unspecific antibodies. All other antibodies did not present any binding for any of the proteins tested.

### Determining potential epitope candidates

To further refine the determination of the binding of each antibody, epitope mapping was performed for Aldo-keto reductase family 1 member A1 (AKR1A1), phosphoribosylglycinamide synthetase (GART) and for one protein of each type of enzyme, namely peptidyl-prolyl isomerase-like 1 (PPIL1) and magnesium dependent phosphatase 1 (MDP1). In addition, the following cancer associated antigens were tested: microtubule associated tumor suppressor candidate 2 (MTUS2), DEAD-box helicase 53 (DDX53), LIM and SH3 domain protein 1 (LASP1), Kinesin family member C3 (KIFC3) and Lamin-A/C (LMNA). It was found that AKR1A1, DDX53, KIFC3 and MDP1 are the most prominent targets (Table 4, Table 5).

**Table 4: Binding patterns found in the microarray analysis. Marked sequences are identical with the potential epitopes found by the bioinformatics analysis.**

| **Antibody** | **potential target** | **binding stretch (SEQ ID NO:)** | | | | | | **potential epitope** |
|---|---|---|---|---|---|---|---|---|
| B4 | DDX53 | **EAKVRIF**GNREMKAK (56) | | | | | | RIFGNREMKAK (SEQ ID NO: 49) |
| | | | KVRIFGNREMKAKAK (57) | | | | | |
| | | | | RIFGNREMKAKAKAA (58) | | | | |
| | | LKSGEKRLIPKPTCR (59) | | | | | | SGEKRLIPKPTCR (SEQ ID NO: 50) |
| | | | SGEKRLIPKPTCRFK (60) | | | | | |
| | | SRGLDLNDVTHVYNY (61) | | | | | | HVYNY (SEQ ID NO: 51) |
| | | | GLDLNDVTHVYNYDF (62) | | | | | |
| | | | | | | | **HVYNYDFPR**NIDVYV (63) | |
| F6 | KIFC3 | ASDWEYTITVSAAEI (64) | | | | | | EYTITVSAAEI (SEQ ID NO: 52) |
| | | | DWEYTITVSAAEIYN (65) | | | | | |
| | | | | EYTITV**SAAEIYNEV** (66) | | | | |
| | | AELGSWSSQEHLEWE (67) | | | | | | SWSSQEHLEWE (SEQ ID NO: 53) |
| | | | LGSWSSQEHLEWEPA (68) | | | | | |
| | | | | SWSSQEHLEWEPACQ (69) | | | | |
| F1 | AKR1A1 | RKTLADLQLEYLDLY (70) | | | | | | LEYLDLY (SEQ ID NO: 54) |
| | | | TLADLQLEYLDLYLM (71) | | | | | |
| | | | | ADLQLEYLDLYLMHW (72) | | | | |
| | | | | | LQLEYLDLYLMHWPY (73) | | | |
| | | | | | | LEYLDL**YLMHWPYAF** (74) | | |
| | | WRYIVPMLTVDGKRV (75) | | | | | | TVDGKRV (SEQ ID NO: 55) |
| | | | YIVPMLTVDGKRVPR (76) | | | | | |
| | | | | VPMLTVDGKRVPRDA (77) | | | | |
| | | | | | MLTVDGKRVPRDAGH (78) | | | |
| | | | | | | TVDGKRVPRDAGHPL (79) | | |

### Bioinformatics approach for epitope recognition

Since it is hypothesized that the target proteins are presented on MHC I, an IEDB analysis was performed, which revealed a set of possible epitopes for each target that could be presented on MHC I. For each target potential epitopes were isolated when comparing the microarray analysis with the predictions and experimental data from IEDB. One potential epitope for AKR1A1 is YLMHWPYAF (aa 110-118; SEQ ID NO: 80) which was detected in the microarray analysis of P3-F1 (Table 4) and also the IEDB predictions suggested an IC50 below 50 nM indicating a high binding potential to MHC I. Additionally, this exact epitope was already experimentally confirmed for patients suffering from brain cancer, adenocarcinoma or leukemia. Two epitope candidates were found for DDX53. The epitopes GESEAKVRIF (aa 87-96; SEQ ID NO: 81) and HVYNYDFPR (aa 538-546; SEQ ID NO: 82) were both found in the microarray for P1-B4 (Table 4) but only the later motif was found to have a predicted IC50 below 50 nM, whereas the first motif is predicted to have an intermediate binding affinity to MHCI. For both epitopes no experimental data is available in the IEDB. Lastly, SAAEIYNEV (aa 573-581; SEQ ID NO: 83) was found as a potential epitope candidate for KIFC3. P1-F6 binds to this epitope in the microarray analysis (Table 4) and also the IEDB prediction presents very low IC50 values around 5 nM for this epitope. Additionally, this epitope was already experimentally confirmed for patients with different kinds of cancer including brain cancer and melanoma.

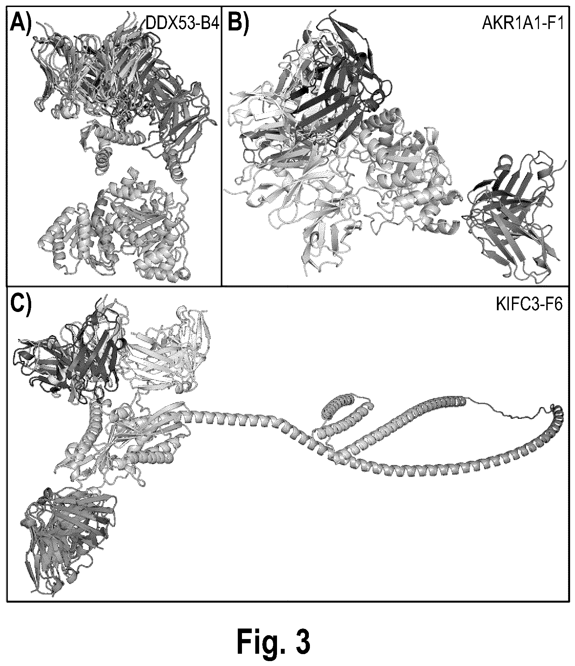

In order to further determine the epitope of the recombinant antibodies, the binding of P1-B4, P1-F6, and P3-F1 to their respective targets (DDX53, KIFC3, AKR1A1) was analyzed using AbAdapt. In Figure 3, the top five clusters are presented for each antibody-antigen pair, whereas the antibodies are colored, and the antigens are shown in grey. In general, but especially for DDX53, it can be recognized that most of the predicted antibody binding sides are in the same region. Besides that, in KIFC3 it looks like there are two regions capable of antibody binding. Interestingly, the highest rated cluster (red) for AKR1A1 and F1 binds on a very different side as all other antibodies. To further evaluate the possible binding epitope for each antibody close interactions for each of the clusters from the antibody-antigen pair were determined. For DDX53 and P1-B4 most of the binding clusters present close interactions for the amino acids of the GESEAKVRIF (aa 87-96; SEQ ID NO: 81) motif. Taken together with the previous results, this suggests that this motif may be the epitope of this target. In the analysis of AKR1A1 and P3-F1, none of the amino acid residues of YLMHWPYAF (aa 110-118; SEQ ID NO: 80) were found to be close interaction partners. However, amino acids in proximity to this motif were close interactors. For KIFC3 and P1-F6, very different interacting amino acids were found in the different clusters. There was also no overlap with previously identified epitopes.

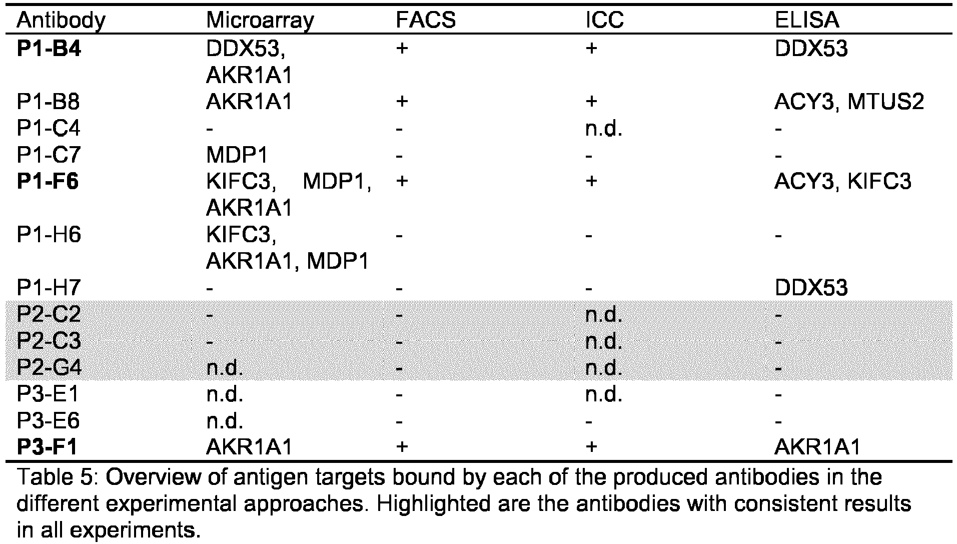

### 4. Conclusion

The aim of the study underlying the invention was to determine whether an intrathecal B cell response in patients with malignant melanoma and meningeal spread can be directed against tumor cells in the CSF and what potential targets can be identified by CSF B cell-derived recombinant antibodies. These antibodies and their corresponding tumor antigens may be extremely useful as they appear to be sufficiently immunogenic to elicit an immune response and may target specific epitopes, thereby reducing off-target effects through B cell receptor selection. In this study, an intrathecal B cell response in 3 patients with malignant melanoma was identified and 13 recombinant antibodies were generated for further analysis (Table 5).

Although antigen detection of antibodies is often difficult due to cross-reactivity and the conformational nature of many antigens, two target antigens were identified in patient 1 and one target antigen in patient 3. It was demonstrated by flow cytometry that the antibodies P1-B4, P1-B8, P1-F6, and P3-F1 bind extracellularly to the Malme-3M cell line, which represents molecular characteristics of the tumor cells of melanoma patients. Further immunostaining in fixed cells showed a cytosolic staining pattern and expression at the edge of the cells. Microarrays of pooled antibodies revealed several potential antigens, which were further selected according to their biological function and tumor association. For the selected antigens AKR1A1, GART, PPIL1, MDP1, MTUS2, DDX53, LASP1, KIFC3 and LMNA, additional epitope mapping by permutation of linear peptides of 13 AA was performed. The antibody P1-B4 could be associated with binding to a potential specific epitope for DDX53, the antibody P1-F6 with an epitope for the antigen KIF3C and the antibody P3-F1 with an epitope for the antigen AKR1A1. Potential epitope sequences were also isolated with bioinformatic simulations of binding patterns between the antibody and the antigen as well as MHC I. For DDX53 the potential epitopes were RIFGNREMKAK (SEQ ID NO: 49), SGEKRLIPKPTCR (SEQ ID NO: 50), and HVYNY (SEQ ID NO: 51), for AKR1A1 LEYLDLY (SEQ ID NO: 54) and TVDGKRV (SEQ ID NO: 55) was found and lastly EYTITVSAAEI (SEQ ID NO: 52) and SWSSQEHLEWE (SEQ ID NO: 53) might be the epitope for KIFC3. Finally, custom ELISA tests were established that confirmed binding of the antibody P1-B4 against DDX53, and P1-F6 against KIF3C in patient 1; serum and CSF of this patient was also tested positive against DDX53 whereas serum was positive against KIF3C only. In patient 3, the inventors could confirm AKR1A1 as a target of antibody P3-F1 with serum also showing positive signal in the ELISA test.

Altogether all three proteins, DDX53, AKR1A1, and KIFC3, are highly interesting and promising targets for an anti-cancer treatment. In addition, tumor antigens recovered by CSF may be extremely useful since they seem to be sufficiently immunogenic to trigger an immune response and may target specific epitopes reducing off-target effects by B cell receptor selection.

### Sequences

Part of the application and disclosure is a sequence listing in WIPO St.26-Forrmat. In the following, all sequences are again listed which are subject invention. In the event of discrepancies between the sequences in the attached sequence listing and the sequences listed in the following, the sequences listed in the following shall take precedence.

### ANTIBODIES

### AMINO ACIDS

### CDR3

Clone P1-B4 heavy chain CDR3 amino acid
   ARSRGIVGSTLDDN (SEQ ID NO: 1)
Clone P1-F6 heavy chain CDR3 amino acid
   AHRPIASSRDGFDY (SEQ ID NO: 2)
Clone P3-F1 heavy chain CDR3 amino acid
   SHHRLTDGRLR (SEQ ID NO: 3)
Clone P1-B4 light chain CDR3 amino acid
   QQYHSTPRT (SEQ ID NO: 4)
Clone P1-F6 light chain CDR3 amino acid
   QQYNGFPFT (SEQ ID NO: 5)
Clone P3-F1 light chain CDR3 amino acid
   QQYDTSPWT (SEQ ID NO: 6)

### CDR2

Clone P1-B4 heavy chain CDR2 amino acid
   ISVYKSDT (SEQ ID NO: 7)
Clone P1-F6 heavy chain CDR2 amino acid
   IYWNDDK (SEQ ID NO: 8)
Clone P3-F1 heavy chain CDR2 amino acid
   VYWDDDK (SEQ ID NO: 9)
Clone P1-B4 light chain CDR2 amino acid
   WAS (SEQ ID NO: 10)
Clone P1-F6 light chain CDR2 amino acid
   KAS (SEQ ID NO: 11)
Clone P3-F1 light chain CDR2 amino acid
   RAS (SEQ ID NO: 12)

### CDR1

Clone P1-B4 heavy chain CDR1 amino acid
   GYTFTSYG (SEQ ID NO: 13)
Clone P1-F6 heavy chain CDR1 amino acid
   GFSLSTNGVS (SEQ ID NO: 14)
Clone P3-F1 heavy chain CDR1 amino acid
   GFSLNTRAVG (SEQ ID NO: 15)
Clone P1-B4 light chain CDR1 amino acid
   QSVLYSSNNRNY (SEQ ID NO: 16)
Clone P1-F6 light chain CDR1 amino acid
   QSVSGW (SEQ ID NO: 17)
Clone P3-F1 light chain CDR1 amino acid
   QSITSSY (SEQ ID NO: 18)

### Full-length clone P1-B4

Heavy chain 
QVQLVESGPEVKKPGASVKVSCKASGYTFTSYGISWVRQAPGQGLEWMGWISVYKSDT
NDAQKFQGRVTMTTDTSTSTAYMELRSLRSDDTAVYYCARSRGIVGSTLDDNWGQGTL
VTVSS (SEQ ID NO: 19)
Light chain

HPQMTQSPDSLAVSLGERATINCKSSQSVLYSSNNRNYLAWYQQKPGQPPKLLIYWAST
RESGVPDRFSGSGSGTDFTLTISSLQAEDVAVYYCQQYHSTPRTFGQGTKVEIK (SEQ
ID NO: 20)

### Full-length clone P1-F6

Heavy chain 
QVPLKESGPALVKPTRTLTLTCTFSGFSLSTNGVSVGWIRQPPGKALDWLALIYWNDDK
RYSPSLKNRLTITKDTSKNLVVLRMTNMDPVDTATYYCAHRPIASSRDGFDYWGQGTLV
TVSS (SEQ ID NO: 21)
Light chain

DIQMTQFPSTLSASVGETVTISCRASQSVSGWLAWYQQKPGKAPKLLMYKASALENGVP
SRFSGSGSGTEFTLTISSLQPDDFATYYCQQYNGFPFTFGPGTKVDIN (SEQ ID NO: 22)

### Full-length clone P3-F1

Heavy chain 
QVQLVESGPTLVKPTQTLTVTCTFSGFSLNTRAVGVGWIRQPPGGALEWLALVYWDDD
KRYSPSLKNRLTITKDTSRNQVVLTLTNVAPVDTGTYYCSHHRLTDGRLRWGQGTLVTV
SS (SEQ ID NO: 23)
Light chain

QVQLVQSPGTLSLSPGERATLSCRASQSITSSYLAWYQQKFGQAPRLLIYRASNRATGIP
DRFSGSGSGTDFTLTISSLESEDFAVYYCQQYDTSPWTFGQGTKVEIK (SEQ ID NO: 24)

### NUCLEIC ACIDS

### CDR3

Clone P1-B4 heavy chain CDR3 nucleic acid
   GCC CGC TCT AGG GGC ATC GTG GGC AGT ACA CTG GAC GAC AAC (SEQ ID NO: 25)
Clone P1-F6 heavy chain CDR3 nucleic acid
   GCC CAC AGG CCC ATC GCC TCC TCC CGA GAC GGC TTC GAC TAC (SEQ ID NO: 26)
Clone P3-F1 heavy chain CDR3 nucleic acid
   AGC CAC CAC AGG CTG ACC GAC GGA AGA CTG AGG (SEQ ID NO: 27)
Clone P1-B4 light chain CDR3 nucleic acid
   CAG CAG TAC CAC TCC ACC CCC AGA ACC (SEQ ID NO: 28)
Clone P1-F6 light chain CDR3 nucleic acid
   CAG CAG TAC AAT GGC TTT CCC TTC ACA (SEQ ID NO: 29)
Clone P3-F1 light chain CDR3 nucleic acid
   CAG CAG TAC GAC ACC TCT CCC TGG ACC (SEQ ID NO: 30)

### CDR2

Clone P1-B4 heavy chain CDR2 nucleic acid
   ATC TCC GTG TAT AAG AGC GAT ACA (SEQ ID NO: 31)
Clone P1-F6 heavy chain CDR2 nucleic acid
   ATC TAC TGG AAC GAC GAC AAA (SEQ ID NO: 32)
Clone P3-F1 heavy chain CDR2 nucleic acid
   GTG TAC TGG GAC GAC GAC AAG (SEQ ID NO: 33)
Clone P1-B4 light chain CDR2 nucleic acid
   TGG GCA TCC (SEQ ID NO: 34)
Clone P1-F6 light chain CDR2 nucleic acid
   AAA GCC TCC (SEQ ID NO: 35)
Clone P3-F1 light chain CDR2 nucleic acid
   AGG GCC TCC (SEQ ID NO: 36)

### CDR1

Clone P1-B4 heavy chain CDR1 nucleic acid
   GGC TAC ACC TTC ACC TCC TAC GGC (SEQ ID NO: 37)
Clone P1-F6 heavy chain CDR1 nucleic acid
   GGC TTC TCC CTG TCC ACC AAC GGC GTG TCC (SEQ ID NO: 38)
Clone P3-F1 heavy chain CDR1 nucleic acid
   GGC TTC TCA CTG AAC ACA AGG GCC GTG GGC (SEQ ID NO: 39)
Clone P1-B4 light chain CDR1 nucleic acid
   CAG TCC GTC CTC TAC TCC TCC AAC AAC CGC AAC TAC (SEQ ID NO: 40)
Clone P1-F6 light chain CDR1 nucleic acid
   CAG TCC GTG TCC GGC TGG (SEQ ID NO: 41)
Clone P3-F1 light chain CDR1 nucleic acid
   CAG TCA ATC ACA TCC TCC TAC (SEQ ID NO: 42)

### Full-length clone P1-B4

Heavy chain 
CAGGTGCAGCTGGTGGAAAGTGGCCCCGAGGTGAAAAAGCCCGGCGCCTCCGTGA
AGGTGTCCTGCAAAGCAAGCGGCTACACCTTCACCTCCTACGGCATCTCCTGGGTGA
GGCAGGCCCCCGGACAGGGACTGGAGTGGATGGGATGGATCTCCGTGTATAAGAG
CGATACAAATGACGCCCAGAAGTTCCAGGGCCGCGTGACAATGACCACCGACACCT
CCACCAGCACCGCTTATATGGAGCTGAGATCCCTGAGGAGCGACGACACCGCCGTG
TACTACTGTGCCCGCTCTAGGGGCATCGTGGGCAGTACACTGGACGACAACTGGGG
CCAGGGCACCCTGGTGACAGTGAGCTCC (SEQ ID NO: 43)
Light chain

CACCCTCAGATGACCCAGTCCCCCGACTCCCTGGCCGTCTCCCTCGGAGAAAGAGC
CACCATCAACTGCAAATCCTCCCAGTCCGTCCTCTACTCCTCCAACAACCGCAACTA
CCTCGCCTGGTACCAGCAGAAACCCGGCCAGCCCCCCAAACTGCTCATCTACTGGG
CATCCACCAGAGAATCCGGCGTGCCCGACCGCTTCTCCGGCTCTGGAAGCGGCACC
GACTTCACCCTGACTATCTCCTCCCTGCAGGCTGAGGACGTCGCCGTTTACTACTGC
CAGCAGTACCACTCCACCCCCAGAACCTTCGGCCAGGGCACCAAGGTGGAAATCAA
A (SEQ ID NO: 44)

### Full-length clone P1-F6

Heavy chain 
CAGGTGCCACTGAAGGAAAGCGGCCCCGCCCTGGTGAAACCCACCAGAACACTGAC
CCTGACCTGCACCTTCTCCGGCTTCTCCCTGTCCACCAACGGCGTGTCCGTGGGCT
GGATCAGGCAGCCCCCCGGAAAAGCCCTGGACTGGCTGGCTCTGATCTACTGGAAC
GACGACAAAAGATACTCCCCCTCCCTGAAGAACCGGCTGACCATCACAAAAGACACC
AGCAAGAACCTGGTGGTGCTGAGGATGACCAACATGGACCCTGTGGACACCGCCAC
CTACTACTGCGCCCACAGGCCCATCGCCTCCTCCCGAGACGGCTTCGACTACTGGG
GACAGGGAACCCTGGTGACCGTGTCCAGC (SEQ ID NO: 45)
Light chain

GACATTCAGATGACCCAGTTTCCCTCCACCCTGTCCGCCTCCGTGGGCGAAACCGT
GACCATCTCCTGCAGGGCCTCCCAGTCCGTGTCCGGCTGGCTGGCTTGGTACCAGC
AGAAACCCGGCAAAGCCCCCAAACTGCTGATGTACAAAGCCTCCGCCCTGGAGAAC
GGCGTGCCCTCACGCTTTAGCGGTTCCGGCTCCGGGACCGAGTTCACCCTGACTAT
CTCCTCACTGCAGCCCGACGACTTCGCCACCTACTACTGCCAGCAGTACAATGGCTT
TCCCTTCACATTCGGTCCCGGCACAAAGGTGGATATTAAC (SEQ ID NO: 46)

### Full-length clone P3-F1

Heavy chain 
CAGGTGCAGCTGGTGGAAAGTGGCCCCACCCTGGTGAAGCCCACACAGACACTGAC
AGTGACATGCACATTCTCCGGCTTCTCACTGAACACAAGGGCCGTGGGCGTGGGAT
 
GGATCAGGCAGCCCCCCGGAGGCGCACTGGAATGGCTGGCTCTGGTGTACTGGGA
CGACGACAAGAGGTACTCCCCTAGTCTGAAGAACAGGCTGACAATCACAAAGGATAC
CAGCAGGAACCAGGTGGTGCTGACACTGACCAACGTGGCTCCCGTGGACACCGGC
ACCTACTACTGCAGCCACCACAGGCTGACCGACGGAAGACTGAGGTGGGGCCAGG
GCACCCTGGTGACAGTGTCCTCT (SEQ ID NO: 47)
Light chain

CAGGTCCAGCTCGTCCAGAGCCCCGGCACACTCTCCCTCTCCCCCGGAGAAAGAGC
CACACTCTCATGCCGGGCCTCCCAGTCAATCACATCCTCCTACCTCGCCTGGTACCA
GCAGAAATTCGGCCAGGCCCCCAGGCTCCTGATCTACAGGGCCTCCAACAGGGCCA
CCGGAATCCCCGACAGGTTTTCCGGCTCCGGCTCCGGAACCGACTTCACCCTGACT
ATCTCCTCCCTCGAGTCTGAGGACTTCGCAGTTTACTACTGCCAGCAGTACGACACC
TCTCCCTGGACCTTCGGCCAGGGTACCAAGGTGGAGATCAAG (SEQ ID NO: 48)

### EPITOPES

### AMINO ACIDS

RIFGNREMKAK (SEQ ID NO: 49)
SGEKRLIPKPTCR (SEQ ID NO: 50)
HVYNY (SEQ ID NO: 51)
EYTITVSAAEI (SEQ ID NO: 52)
SWSSQEHLEWE (SEQ ID NO: 53)
LEYLDLY (SEQ ID NO: 54)
TVDGKRV (SEQ ID NO: 55)
YLMHWPYAF (SEQ ID NO: 80)
GESEAKVRIF (SEQ ID NO: 81)
HVYNYDFPR (SEQ ID NO: 82)
SAAEIYNEV (SEQ ID NO: 83)

### BINDING STRETCHES

### AMINO ACIDS

EAKVRIFGNREMKAK (SEQ ID NO: 56)
KVRIFGNREMKAKAK (SEQ ID NO: 57)
RIFGNREMKAKAKAA (SEQ ID NO: 58)
LKSGEKRLIPKPTCR (SEQ ID NO: 59)
SGEKRLIPKPTCRFK (SEQ ID NO: 60)
SRGLDLNDVTHVYNY (SEQ ID NO: 61)
GLDLNDVTHVYNYDF (SEQ ID NO: 62)
HVYNYDFPRNIDVYV (SEQ ID NO: 63)
ASDWEYTITVSAAEI (SEQ ID NO: 64)
DWEYTITVSAAEIYN (SEQ ID NO: 65)
EYTITVSAAEIYNEV (SEQ ID NO: 66)
AELGSWSSQEHLEWE (SEQ ID NO: 67)
LGSWSSQEHLEWEPA (SEQ ID NO: 68)
SWSSQEHLEWEPACQ (SEQ ID NO: 69)
RKTLADLQLEYLDLY (SEQ ID NO: 70)
TLADLQLEYLDLYLM (SEQ ID NO: 71)
ADLQLEYLDLYLMHW (SEQ ID NO: 72)
LQLEYLDLYLMHWPY (SEQ ID NO: 73)
LEYLDLYLMHWPYAF (SEQ ID NO: 74)
WRYIVPMLTVDGKRV (SEQ ID NO: 75)
YIVPMLTVDGKRVPR (SEQ ID NO: 76)
VPMLTVDGKRVPRDA (SEQ ID NO: 77)
MLTVDGKRVPRDAGH (SEQ ID NO: 78)
TVDGKRVPRDAGHPL (SEQ ID NO: 79)

## Claims

1. An antibody or fragment thereof specifically binding to meningeosis carcinoma-specific antigen (MC-specific antigen), **characterized in** comprising
- as heavy chain variable domain a CDR3 region, said CDR3 region has an amino acid sequence being at least 90 % identical to one selected from those in SEQ ID NO: 1, 2 or 3, and/or
- as light chain variable domain a CDR3 region, said CDR3 region has an amino acid sequence being at least 90% identical to one selected from those in SEQ ID NO: 4, 5 or 6.

2. The antibody or fragment of claim 1, **characterized in that**
- the heavy chain variable domain comprises a CDR2 region, said CDR2 region has an amino acid sequence being at least 90 % identical to one selected from those in SEQ ID NO: 7, 8 or 9, and/or
- the light chain variable domain comprises a CDR2 region, said CDR2 region has an amino acid sequence being at least 90 % identical to one selected from those in SEQ ID NO: 10, 11, or 12.

3. The antibody or fragment of claim 1 or 2, **characterized in that**
- the heavy chain variable domain comprises a CDR1 region, said CDR1 region has an amino acid sequence being at least 90 % identical to one selected from those in SEQ ID NO: 13, 14, or 15, and/or
- the light chain variable domain comprises a CDR1 region, said CDR1 region has an amino acid sequence being at least 90 % identical to one selected from those in SEQ ID NO: 16, 17, or 18.

4. The antibody or fragment of any of claims 1-3, **characterized in** comprising
- the heavy chain variable domains
- CDR3 region having an amino acid sequence being at least 90 % identical to said in SEQ ID NO: 1 and/or
- CDR2 region having an amino acid sequence being at least 90 % identical to said in SEQ ID NO: 7 and/or
- CDR1 region having an amino acid sequence being at least 90 % identical to said in SEQ ID NO: 13 and/or
- the light chain variable domains
- CDR3 region having an amino acid sequence being at least 90 % identical to said in SEQ ID NO: 4 and/or
- CDR2 region having an amino acid sequence being at least 90 % identical to said in SEQ ID NO: 10 and/or
- CDR1 region having an amino acid sequence being at least 90 % identical to said in SEQ ID NO: 16.

5. The antibody or fragment of any of claims 1-3, **characterized in** comprising
- the heavy chain variable domains
- CDR3 region having an amino acid sequence being at least 90 % identical to said in SEQ ID NO: 2 and/or
- CDR2 region having an amino acid sequence being at least 90 % identical to said in SEQ ID NO: 8 and/or
- CDR1 region having an amino acid sequence being at least 90 % identical to said in SEQ ID NO: 14 and/or
- the light chain variable domains
- CDR3 region having an amino acid sequence being at least 90 % identical to said in SEQ ID NO: 5 and/or
- CDR2 region having an amino acid sequence being at least 90 % identical to said in SEQ ID NO: 11 and/or
- CDR1 region having an amino acid sequence being at least 90 % identical to said in SEQ ID NO: 17.

6. The antibody or fragment of any of claims 1-3, **characterized in** comprising
- the heavy chain variable domains
- CDR3 region having an amino acid sequence being at least 90 % identical to said in SEQ ID NO: 3 and/or
- CDR2 region having an amino acid sequence being at least 90 % identical to said in SEQ ID NO: 9 and/or
- CDR1 region having an amino acid sequence being at least 90 % identical to said in SEQ ID NO: 15 and/or
- the light chain variable domains
- CDR3 region having an amino acid sequence being at least 90 % identical to said in SEQ ID NO: 6 and/or
- CDR2 region having an amino acid sequence being at least 90 % identical to said in SEQ ID NO: 12 and/or
- CDR1 region having an amino acid sequence being at least 90 % identical to said in SEQ ID NO: 18.

7. The antibody of fragment thereof or any of the preceding claims comprising
- a heavy chain having an amino acid sequence being at least 90 % identical to said in SEQ ID NO:19, and/or
- a light chain having an amino acid sequence being at least 90 % identical to said in SEQ ID 20.

8. The antibody of fragment thereof or any of the preceding claims comprising
- a heavy chain having an amino acid sequence being at least 90 % identical to said in SEQ ID NO:21, and/or
- a light chain having an amino acid sequence being at least 90 % identical to said in SEQ ID 22.

9. The antibody of fragment thereof or any of the preceding claims comprising
- a heavy chain having an amino acid sequence being at least 90 % identical to said in SEQ ID NO:23, and/or
- a light chain having an amino acid sequence being at least 90 % identical to said in SEQ ID 24.

10. The antibody or fragment thereof of any of the preceding claims, **characterized in that** the MC-specific antigen is selected from the group consisting of DDX53, KIF3C, and AKR1A1.

11. An antibody or fragment thereof specifically binding to MC-specific antigen, **characterized in that** it specifically binds to an epitope comprising an amino acid sequence being at least 90% identical to one selected from those in SEQ ID NOS: 49-55.

12. An antibody or fragment thereof configured for a specific binding to meningeosis carcinoma-specific antigen for use in the prophylaxis and/or treatment of cancer, preferably melanoma, further preferably melanoma with leptomeningeal spread, highly preferably melanoma with meningeosis carcinomatosa.

13. A pharmaceutical composition **characterized in** comprising an antibody or fragment thereof according to any of claims 1-12.

14. A nucleic acid, preferably a vector, encoding an antibody or fragment thereof according to any of claims 1-13.

15. A prokaryotic or eukaryotic host cell comprising the nucleic acid of claim 14.
